# EUROPEAN PATENT SPECIFICATION

(11) **EP 1 345 597 B1**
(45) Date of publication and mention of the grant of the patent: **03.10.2007**
(21) Application number: 01995691.1
(22) Date of filing: 17.12.2001
(51) Int. Cl.: A61K 9/51, A61K 38/09

(54) **AMPHIPHILIC LIPID NANOPARTICLES FOR PEPTIDE AND/OR PROTEIN INCORPORATION**
AMPHIPHILE LIPID-NANOPARTIKEL ZUR PEPTID- UND/ODER PROTEININKORPORATION
NANOPARTICULES LIPIDIQUES AMPHIPHILES DESTINEES A L'INCORPORATION DE PEPTIDES ET/OU PROTEINES

(30) Priority: 27.12.2000 EP 00128555; 26.10.2001 EP 01125742
(43) Date of publication of application: 24.09.2003
(73) Proprietor: ARES TRADING S.A., 1170 Aubonne (CH)
(72) Inventor: DEL CURTO, Maria Dorly, I-27050 Corvino San Quirico (IT); CHICCO, Daniela, I-10010 Caravino (IT); ESPOSITO, Pierandrea, I-10015 Ivrea (IT)
(74) Representative: Merck Serono International S.A. Intellectual Property
(86) International application number: PCT/EP2001/014877
(87) International publication number: WO 2002/051390

(56) References cited:
- DE-A- 19 819 273
- MÜLLER RH ET AL.: "Solid lipid nanoparticles (SLN) for controlled drug delivery - a review of the state of the art" EUROPEAN JOURNAL OF PHARMACEUTICS AND BIOPHARMACEUTICS, vol. 50, July 2000 (2000-07), pages 161-177, XP002169638 ISSN: 0939-6411
- MOREL S ET AL.: "Incorporation in lipospheres of (D-Trp-6)LHRH" INTERNATIONAL JOURNAL OF PHARMACEUTICS (AMSTERDAM), vol. 105, no. 2, 1994, pages R1-R3, XP001009426 ISSN: 0378-5173

## Description

### Field of the Invention

This invention relates to lipid nanoparticles consisting of lipids enriched In amphiphilic components, which promote the incorporation of peptides and/or proteins, process for obtaining them as well as use thereof.

### Background of the invention

Drug delivery systems, which have been investigated for many years, include microparticles; liposomes oil-in-water (O/W) emulsions and nanoparticles based on synthetic polymers or natural macromolecules.

The use of solid lipids as matrix material for drug delivery is well known from lipid pellets for oral drug delivery (e.g. Mucosolvan ® retard capsules). The production of lipid microparticles by spray congealing was described by Speiser at the beginning of the eighties (Speiser and al., Pharm. Res. 8 (1991) 47-54) followed by lipid nanopellets for peroral administration (Speiser EP 0167825 (1990)). Basically, lipids, which can be used, are well tolerated by the body (e.g. glycerides composed of fatty acids which are present in the emulsions for parenteral nutrition).

Phospholipid vesicles rediscovered as "liposomes" in 1965 by Bagham found their way to the cosmetic market in 1986 (J.E. Diederichs and al., Pharm. Ind. 56 (1994) 267-275).

Regarding to polymeric microparticles, their number on the market is limited, and there was only a limited increase in the number of microparticulate products. The situation is even worse for polymeric nanoparticles, after more than 30 years of research, this delivery system practically does not exist on the market. There are quite well known reasons for this, among which two should be highlighted: the cytotoxicity of polymers and the lack of a suitable large-scale production method. Polymers accepted for use, as implants are not necessarily also of good tolerability in the form of nanoparticles. In the nanometer size range and having a size of a few micrometers, the polymer can be internalised by cells (e.g. macrophages) and degradation inside the cell can lead to cytotoxic effect e.g. as reported for polyester polymers (Int.J.Pharm. 30 (1986) pp. 215-220, A.Smith).

Considering the limitations of conventional drug carriers, such as liposomes, lipid emulsions, nanoparticles and microspheres, there is an obvious demand for a carrier system for the controlled delivery of bioactive substances such as peptides or proteins to circumvent the drawbacks of traditional systems particularly with regard to preparation, stability and toxicity.

Lipid nanoparticles represent an alternative carrier system to traditional colloidal carriers such as emulsions, liposomes and polymeric micro- and nanoparticles and possess the properties mentioned hereinafter:
- biodegradability and non-toxicity;
- the ability to incorporate poorly water-soluble substances;
- improved chemical and physical stability;
- the possibility to prepare a dry storage formulation;
- controlled release of incorporated substances.

It is also well known that lipid nanoparticles are characterized as lipidic particles of a solid physical state in the nanometer size range combining the advantages of other drug delivery systems such as the followings:
- solid state of the lipid matrix allows prolonged drug release and
- protects incorporated ingredient against chemical degradation,
- the lipids are basically well tolerated (low cellular and systemic toxicity),
- large scale production is possible by high pressure homogenization, similar to emulsions and liposomes.

Many different drugs have been incorporated in lipid nanoparticles, and its loading capacity has been judged to evaluate the suitability of a drug carrier system. Westesen et al. studied the incorporation of drugs for ubidecarenone (J.Control.Release 48 (1997) pp. 223-236). Several kind of drugs have been described as being able to be incorporated in lipid nanoparticles for example tetracaïne or etomidate (J.Microencapsulation, vol.16, no.2 (1999) pp.205-213), Vitamine E palmitate (Proc. Int. Symp. Control. Release Bioact. Mater. 25 (1998) pp. 433-434), cyclosporin (WO. 99/56733 (1999)), timolol (S.T.P.Pharma Sciences, 6 (1992) 514-518, Gasco et *al*), doxorubicine and idarubicine (Int.J.Pharm. 89 (1993), Gasco et *al*.). However, little has been studied regarding the incorporation of peptides into lipid nanoparticles, as for example Müller et al. with lysozyme-loaded lipid nanoparticles (Int. J. Pharm. 149 (1997) pp.255-265).

Until now, many researchers have prepared lipid nanoparticles as colloidal carriers for the controlled delivery of drugs. Domb et al. (WO 91/07171) produced lipospheres as carriers of drugs and other bioactive agents. By using ultrasonication, Speiser et al (Ger. Offen. DE 3421468 (1985)) obtained lipid nanopellets (80-800 nm) constituted mainly of fatty acids and their esters with glycerol. Sjösrtöm and Bergenstahl obtained lipid nanoparticles by precipitation in solvent emulsions (Int.J.Pharm., 88 (1993) pp. 53-62). Siekmann and Westesen (Pharm.Pharmacol.Lett. 1 (1992) pp. 123-126), Westesen et al (Int.J.Pharm.93 (1993) pp. 189-199) and Müller et al (J.Controlled Re/., 30 (1994) pp. 83-96) produced lipid nanoparticles by high pressure homogenization of melted lipids dispersed in an aqueous surfactant solution.

With regard to peptide and/or protein loading when using lipid nanoparticles as drug delivery system, several problems still remain:
- poor solubility properties regarding said loaded drug,
- low stability of the loaded drug,
- low interactions between drug and lipid matrix,
- enzymatic and chemical degradation,
- bad absorption through the gastrointestinal tract.

DE 198 19 273 A discloses solid lipid particles comprising Ciclosparin and optionally a surfactant.

### Description of the invention:

The present invention provides a new type of lipid nanoparticles comprising amphiphilic lipids suitable for the incorporation of peptides and/or proteins, in order to provide their controlled release, once they have been administered.

In particular, the main object of the invention is to provide a new type of lipid nanoparticles comprising a drug, a lipid matrix and a surfactant characterized in that said drug is a peptide or a protein and said lipid matrix has a monoglyceride content which is at least 70% w/w, the percentage being based on the weight of the lipid matrix.

To obtain an enhanced incorporation of said peptides and/or proteins in the lipid matrix, several lipids with different hydrophilic/hydrophobic characteristics and chemical compositions have been screened, such as for example tri-, di- and mono-glycerides, PEG- or PPG-glycerides, saccharide-glycerides, fatty acids and mixture thereof.

Surprisingly, it has been observed that the maximum peptide and/or protein loading can be obtained by using a lipid matrix containing a high monoglyceride content, which confers amphiphilic properties to the lipid nanoparticles. It has been found that the monoglyceride content of said lipid matrix amount should be at least 70% w/w, preferably from 75 to 99% w/w.. Therefore, according to the present invention, any of the above-mentioned lipids or any mixture of one or more of them may be used, provided that the total amount of mono-glyceride content is at least 70%, as explained above.

According to a preferred embodiment of the invention the lipid matrix has a melting peak at least 65°C, more preferably at least 70°C. The melting peak of the lipid matrix may easily be determined by any method known in the art. One example of these methods is Differential Scanning Calorimetry (DSC).

Moreover, according to another preferred embodiment of the invention, the lipid matrix has a high crystalline content. The crystalline content can be determined by any method known in the art. For example, experiments of DSC can allow to establish qualitatively whether the lipid matrix has a high crystalline content or is polymorphic. In fact, the DSC plot of a polymorphic lipid matrix will show mutiple low and broad melting peaks, whereas the DSC plot a lipid matrix with a high crystalline content will show a high and sharp peak.

The drug-loaded lipid nanoparticles according to the invention are stabilized by compounds such as ionic or non-ionic surfactants. Suitable surfactants include, but are not limited to, the following examples: synthetic phospholipids, their hydrogenated derivatives and mixtures thereof, sphingolipids and glycosphingolipids, saturated or unsaturated fatty acids, fatty alcohols, polyoxyethylene-polyoxypropylene copolymers, ethoxylated fatty acids as well as esters or ethers thereof, dimyristoyl phosphatidyl choline, dimyristoyl phosphatidyl glycerol or a combination of two or more of the above mentioned. A preferred surfactant according to the invention is the dimyristoyl phosphatidyl glycerol.

Said lipid nanoparticles are further, optionally, stabilized by at least one co-surfactant selected in the group comprising or consisting of butanol, butyric acid, hexanoic acid, sodium cholate, sodium taurocholate and sodium glycocholate, more particularly sodium cholate.

Lipid nanoparticles of the invention may also include other excipients, such as polymers having bioadhesive or absorption enhancing properties and selected from the group comprising or consisting of acrylic polymers (Carbopol®, Polycarbophil, Noveon®), medium chain fatty acids and polyethylene glycols. Preferred excipients are the above-mentioned acrylic polymers.

All of the lipid matrix, the surfactant, the co-surfactant and the other excipients are intended to be pharmaceutically acceptable.

Typically any therapeutically effective peptide or protein may be incorporated into the lipid nanoparticles of the invention. Most of the therapeutically useful proteins may be grouped into 3 classes:
- regulatory factors including hormones, cytokines, lymphokines, chemokines, their receptors and other regulatory factors of cellular growth and metabolism comprising enzymes;
- blood products including serum-derived blood factors and enzymatic fibrinogen activators;
- monoclonal antibodies.

According to an embodiment of the invention, suitable proteins or peptides as above-mentioned include, but are not limited to, the following examples: AAT, UK, PUK, streptokinase, tPA, SOD, insulin, GH, GRF, ANF, GnRH, LHRH analogs, erythropoietin, granulocyte CSF, granulocyte macrophage CSF, Interleukin-1, Interleukin-2, Interleukin-3/multipotential CSF, Interleukin-4, Interleukin-5 (or Eosinophil-CSF), Interleukin-6, Interleukin-7, Interleukin-8, Interleukin-9, Interleukin-10, Interleukin-11, interferon-α, interferon-β, interferon-γ, Leukemia inhibitory factor Macrophage CSF,TNF, Stem cell factor as well as receptors thereof.

According to a preferred embodiment of the invention, said protein or peptide is selected from the group consisting of Interleukin-6, Interferon-α, Interferon-β, Interferon-γ, GnRH, LHRH analogs, GH, GRF, gonadotropins (like FSH, LH and hCG) and TNF receptors or soluble fragments thereof.

More preferably the peptide is selected from the group consisting of LHRH analogs, and more particularly a decapeptide acting as LHRH antagonist.

In a particularly preferred embodiment of the present invention, a non-limiting list of said peptides includes the following compounds:
- Abarelix (disclosed in WO 9640757), acts as LHRH antagonist and is defined by the formula hereinafter:
   D-Alaninamide, N-acetyl-3-(2-naphthalenyl)-D-Ala-4-Cl-D- Phe-3-(3-pyridinyl)-D-Ala-L-Ser-N-methyl-L-Tyr-D-Asn-L-Leu-N6-(1-methylethyl)-L-Lys-L-Pro.
- Antarelix (disclosed in WO 9219651), acts as LHRH antagonist and is defined by the following formula:
   D-Alaninamide, N-acetyl-3-(2-naphthalenyl)-D-Ala-4-Cl-D- Phe-3-(3-pyridinyl)-D-Ala-L-Ser-L-Tyr-N6-(aminocarbonyl)- D-Lys-L-Leu-N6-(1-methylethyl)-L-Lys-L-Pro.
- Azaline B (disclosed in US 5296468), acts as GnRH antagonist and is defined by the following formula:
   D-Alaninamide, N-acetyl-3-(2-naphthalenyl)-D-Ala-4-Cl-D- Phe-3-(3-pyridinyl)-D-Ala-L-Ser-4-[(5-amino-1H-1,2,4-triazol- 3-yl)amino]-L-Phe-4-[(5-amino-1H-1,2,4-triazol-3-yl)amino]-D-Phe-L-Leu-N6-(1-methylethyl)-L-Lys-L-Pro.
- Ganirelix (disclosed in EP 277829), acts as LHRH antagonist and is defined by the following formula:
   D-Alaninamide, N-acetyl-3-(2-naphthalenyl)-D-Ala-4-Cl-D- Phe-3-(3-pyridinyl)-D-Ala-L-Ser-L-Tyr-N6- [bis(ethylamino)methylene]-D-Lys-L-Leu-N6-[bis(ethylamino) methylene]- L-Lys-L-Pro.

In a more preferred embodiment of the present invention, said peptide acting as LHRH antagonist is a specific decapeptide named Antide. This decapeptide (N-Ac-D-2-Nal, D-pCIPhe, D-3-Pal, NicLys, D-NicLys, Ilys, D-Ala, NH₂) has an impressive antiovulatory activity as well as LHRH antagonistic properties and has already been described (EP 377665 and US 5470947) as acting directly on the hormonal metabolism in a woman.

Another particular preferred peptide acting as LHRH antagonist is another decapeptide named Cetrotide, (whose INN is Cetrorelix disclosed EP 299402) having the following formula:D-Alaninamide, N-acetyl-3-(2-naphthalenyl)-D-Ala-4-Cl-D-Phe-3-(3-pyridinyl)-D-Ala-L-Ser-L-Tyr-N5-(aminocarbonyl)-D-ornithyl-L-Leu-L-Arg-L-Pro.

Hence, it is herein reported that the peptide- or protein-loaded lipid nanoparticles are indeed suitable for being used as a medicament, for the preparation of a pharmaceutical composition. In the preferred case, where the peptide is a decapeptide acting as LHRH antagonist, the pharmaceutical composition will be useful for the modulation of the hormonal metabolism in a mammal or for the treatment or prevention of disorders associated with abnormal activity of the hormonal metabolism in a woman. More specifically, for the treatment or prevention of disorders associated with abnormal activity of the LHRH pathway. In this specific case, peptide-loaded lipid nanoparticles are useful for the treatment of hormonal diseases, pathological states or contraceptive actions in which antagonizing of LHRH play a major role, such as contraceptive agent for inhibiting the ovulation in mammal or inhibiting the growth of hormone-dependent tumors, or the testosterone production in a mammal. Peptide-loaded lipid nanoparticles could be employed alone or in combination with other pharmaceutical agents.

When employed as pharmaceuticals, peptide- or protein-loaded lipid nanoparticles of the present invention are typically administered in the form of a pharmaceutical dosage form. Hence, pharmaceutical compositions comprising peptide- or protein-loaded lipid nanoparticles and pharmaceutical excipients, such as diluents, antioxidizing agents, surfactants, co-surfactants, viscosizing agents, antimicrobials, cryo-protectants are also in the scope of the present invention. Such composition can be prepared in a manner well known in the pharmaceutical art. Generally, the peptide- or protein-loaded lipid nanoparticles of the present invention are administered in a therapeutically effective amount. The amount actually administered will typically be determined by a physician, in the light of the relevant circumstances, including the condition to be treated, the chosen route of administration, the age, weight, and response of the individual patient, the severity of the patient's symptoms, and the like.

The pharmaceutical compositions of these inventions can be administered by a variety of routes including oral, intravenous, subcutaneous, intramuscular, intraarterial, intraperitoneal, dermal, sublingual, rectal, buccal, vaginal, nasal or pulmonary routes. The oral route of administration is the preferred one according to the invention.

Depending on the intended route of delivery, the compounds can be formulated either as liquid or as solid forms. The compositions for oral administration can take the form of bulk liquid solutions or suspensions, or bulk powders.

Liquid forms suitable for oral administration may include a suitable aqueous or nonaqueous vehicles together with buffers, suspending and dispensing agents, colorants, flavors and the like.

Solid forms may include, for example, any of the following ingredients, or compounds of a similar nature: a binder such as microcrystalline cellulose, gum tragacanth or gelatine; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable carriers known in the art.

A further object of the present invention is a process for preparing the lipid nanoparticles loaded with a peptide or a protein, which have been set out above.

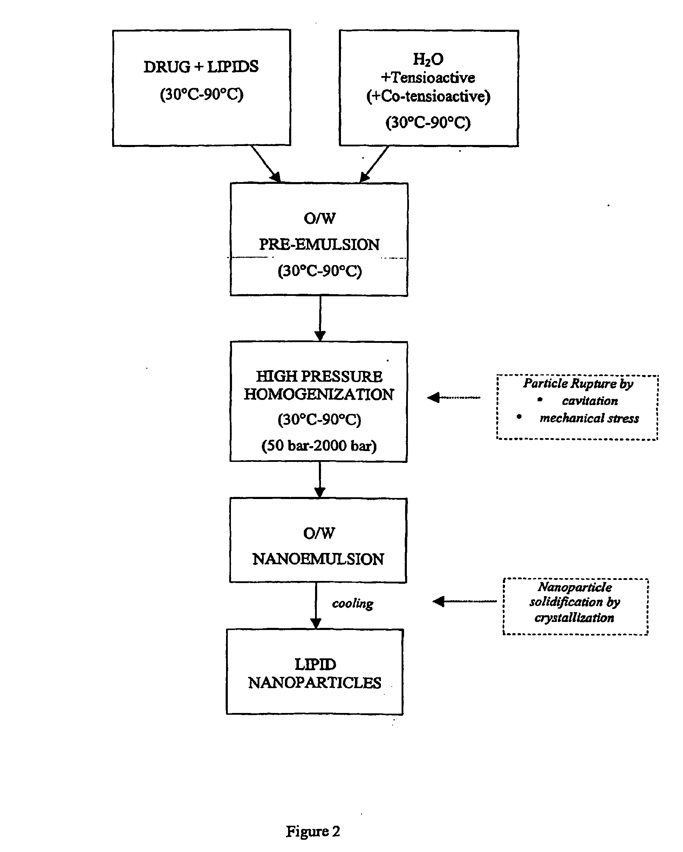

According to a preferred method of production, peptide- or protein-loaded lipid nanoparticles of the present invention can be prepared by a process comprising the following steps (see Figure 2):
1. incorporation of the drug into the lipid phase and dissolution of the surfactant and, optionally, the co-surfactant in the aqueous phase, under controlled heating conditions
2. mixing of the lipid phase with the aqueous phase;
3. Homogenization at High Pressure of the obtained pre-emulsion;
4. cooling down the nano-emulsion under accurately controlled temperature conditions;
5. optionally, eliminating the water.

Each of the above-mentioned steps is detailed hereinafter:

### First step:

Said peptide and/or protein is incorporated into lipid matrices by well-known co-melting technique. In particular, the lipid is first melted in a thermostated bath, at a proper temperature, depending on the lipid used. Said peptide and/or protein is then added stepwise to the molten lipid under constant stirring.

The drug can be also incorporated into the lipid matrix using the "solvent-stripping" technique. The drug and the lipid are first co-solubilized at a suitable temperature in a proper solvent, which is subsequently stripped at proper conditions (under vacuum, at suitable temperature).

In this first step the matrix can be modified with additional excipients, such as phospholipids, with tensioactive properties and hydrophilicity that can further promote drug incorporation into the matrix. Phospholipids are commonly used in pharmaceutical industry for parenteral and enteral emulsion preparation, as well as for stabilization of suspensions, spray and aerosols, and for liposome production. Moreover, these excipients could have a beneficial effect on drug absorption.

Within the first step surfactants and optionally co-surfactants are dissolved in the aqueous phase. The surfactant has a stabilizing effect on the emulsion/suspension by reducing the interfacial tension between lipid and water phase.

The co-surfactant acts also as a stabilizer, since is thought to be placed at the interface between the two phases, thus conferring a charge to the particle surface and preventing particle coalescence.

When employing "solvent-stripping" technique for drug incorporation into the lipid matrix, the solvent used is selected from the group consisting of water, ethanol, propanol, benzyl alcohol, isopropanol, or a mixture thereof, and more particularly benzyl alcohol.

### Second step:

The aqueous and lipid phases, kept at the same temperature (between 30°C and 90°C, particularly 50°C and 85°C, more particularly 65°C and 85°C), are vigorously mixed to obtain a pre-emulsion.

### Third step:

High Pressure Homogenization is applied at a temperature between 30°C and 90°C, with a pressure comprised between 50 bar and 2000 bar, particularly between 500 bar and 1800 bar, more particularly of between 1000 and 1500 bar. During this step, particle rupture occurs by cavitation and mechanical stress leading to a nano-emulsion.

### Fourth step:

The obtained nano-emulsion is cooled down at controlled temperature conditions. Solid lipid nanoparticles appear after crystallisation of the lipid.

The steps above-mentioned are carried out using are carried out with a pH range of between 1 to 9, particularly of between 5 to 7.

At this stage the Lpid nanoparticles of the invention are obtained in solid phase, but in an aqueous suspension.

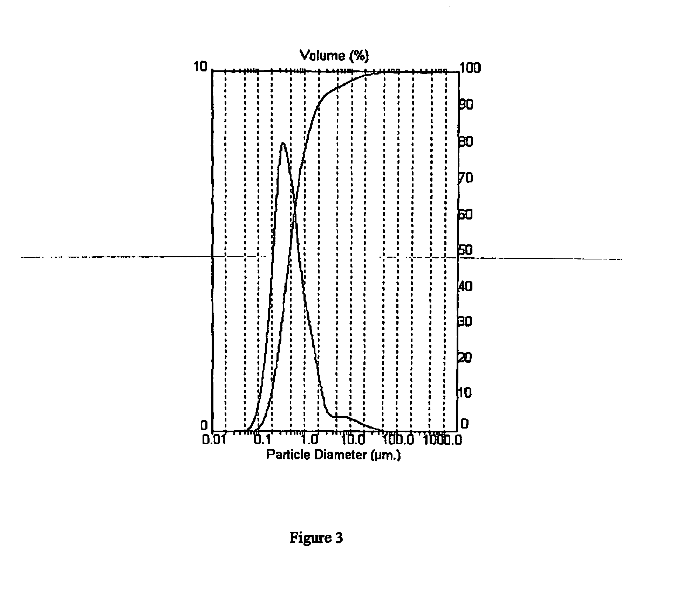

The size (mean diameter) of said nanoparticles within an lipid nanoparticles formulation has a bell-shaped profile (frequency vs. size log plot, see Figure 3) that covers a range from few nm (10 nm) to about 10 µm. Therefore, the size of a population of particles is better described by D (v, 0.1), D (v, 0.5) and D (v, 0.9) parameters, which define the size distribution of the population as follows:
- D (v, 0.1) = 10% (in volume) of the particles have a size below this value
- D (v, 0.5) = 50% (in volume) of the particles have a size below this value
- D (v, 0.9) = 90% (in volume) of the particles have a size below this value.

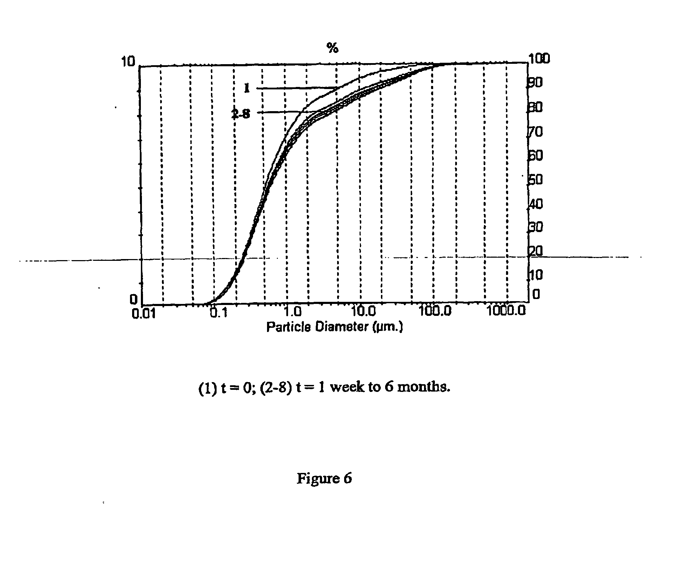

For the most promising lipid nanoparticles formulations we selected, the size parameters are such as:
- D (v, 0.1) = within the range 0.15 µm - 0.20 µm
- D (v, 0.5) = within the range 0.30 µm - 0.60 µm
- D (v, 0.9) = within the range 1 µm - 10 µm
as it can be seen from data shown in Table 3 and from the Figure 6, where overlayed LD volume undersize curves of a lipid nanoparticles formulation analyzed at different times up to 6 months are shown.

The composition of the lipid nanoparticles aqueous formulations obtained according to a preferred embodiment of the present invention is described hereinafter:

| **Peptide or Protein) (% w/w)** | **lipid phase (% w/w)** | **Surfactant (% w/w)** | **Co-surfactant (% w/w)** | **Other excipients (i.e, absorption enhancers/ bioadhesives)** | **H₂O (% w/w)** |
|---|---|---|---|---|---|
| 0,1 to 25 | 2 to 50 | 0,1 to 7 | 0 to 2 | 0.001 to 7 | up to 100 |

According to a preferred composition of the invention, peptide or protein content is from 0,1 to 20 % w/w, particularly from 0,1 to 0,5 % w/w, the percentage being based on the weight of the final aqueous formulation.

In a preferred embodiment of the invention, the lipid matrix content is from 2 to 50 % w/w, particularly from 5 to 40 % w/w, more particularly from 8 to 30 % w/w, the percentage being based on the weight of the final aqueous formulation.

According to another preferred composition of the invention, surfactant content is from 1 to 5 % w/w, particularly from 2 to 4 % w/w, the percentage being based on the weight of the final aqueous formulation.

According to a particularly preferred composition of the invention, co-surfactant content is from 0 to 1 % w/w, particularly from 0,2 to 0,8 % w/w, tthe percentage being based on the weight of the final aqueous formulation.

According to a particularly preferred composition of the invention, absorption enhancing excipient content is from 0 to 5 % w/w, more particularly from 0,2 to 1 % w/w, the percentage being based on the weight of the final aqueous formulation.

According to a particularly preferred composition of the invention, bioadhesive excipient content is from 0 to 0,05 % w/w, particularly from 0,005 to 0.05 % w/w and more particularly from 0,005 to 0.02 % w/w, the percentage being based on the weight of the final aqueous formulation.

Quite surprisingly, peptide-/protein- loaded lipid nanoparticles according to the present invention showed a satisfactory stability (6 months and more) with unchanged encapsulation efficiency (see Fig.6).

A comparison between Compritol Lipid Nanoparticles according to the invention loaded with cyclosporin and Imwitor 900 Lipid Nanoparticles loaded with cyclosporin (as depicted in DE198119273) shows a better stability of the composition regarding to the size (see Fig.4 and Fig.5)

### Fifth Step (optional)

If the lipid nanoparticles are thought to be employed for pharmaceutical compositions in solid dosage forms, the water of the aqueous suspension has to be eliminated. This may be carried out by any technique known in the art, for example by filtration or ultra-filtration or by freeze-drying.

This paragraph provides abbreviations and definitions of the various biological and analytical terms as well as abbreviations used throughout this patent application and are intended to apply uniformly throughout the specification and claims unless and otherwise expressly set out.
"amphiphilic" refers to a compound having affinity for two different environments - for example a molecule with hydrophilic (polar) and lipophilic (non-polar) regions. Detergents are classic examples.
"AAT" refers to α-1-antitrypsin
"ANF" refers to Atrial Natriuretic Factor
"Antide", for which Iturelix is the proposed INN, refers to the following decapeptide:
N-Ac-D-2-Nal, D-*p*CIPhe, D-3-Pal, Ser, NicLys, D-NicLys, Leu, Ilys, Pro, D-Ala, NH₂. wherein:
   "2-Nal" refers to 3-(2-naphtyl)alanine
   "Ilys" refers to N-isopropyllysine
   "NicLys" refers to N-nicotynoyllysine
   "3-Pal" refers to 3-(3-pyridyl)alanine
   "DSC" refers to Differential Scanning Calorimetry
   "ΔH" refers to enthalpy variation
   "DMPC" refers to DiMyristoyl Phosphatidyl Choline
   "DMPG" refers to DiMyristoyl Phosphatidyl Glycerol
   "FACTOR VIII" refers to a glycoprotein containing 2331 amino acids
   "FSH" refers to Follicular Stimulating Hormone.
   "GH" refers to Growth Hormone
   "Glycerides" is intended to mean glycerol esters of C₄-C₃₀ saturated or unsaturated fatty acids
   "GRF" refers to Growth hormone Releasing Factor
   "GnRH" refers to Gonadotropine Releasing Hormone
   "HPH" refers to High Pressure Homogenization
   "LD" refers to Laser Diffractometry
   "LHRH" refers to Luteinizing Hormone Releasing Hormone
   "Lipid", according to the present invention refers to a substance that is poorly soluble in water but is soluble in organic solvents. According to the present invention, lipids include fatty acids, mono- di- and tri-glycerides, phopholipids, PEG-glycerides, saccharide-glycerides or waxes and any mixture thereof
   "LN" refers to lipid nanoparticles
   "m.p." refers to melting point
   "Monoglycerides" refers to compounds obtained applying esterification by fatty acid of one of the glycerol alcohol functions such as shown hereinafter: 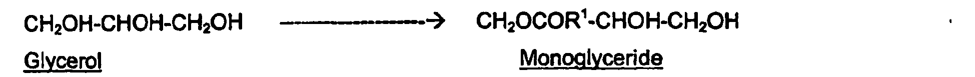
   wherein R¹ is a C₄-C₃₀ saturated or unsaturated hydrocarbon chain;
   or by partial hydrolysis of triglycerides.
   "nanoparticles" refers to particles whose average diameter is comprised in a range from 1 nm to 3000 nm.
   "PCS" refers to Photon Correlation Spectroscopy.
   "PEG" refers to Polyethyleneglycol.
   "Peptide" means a polyamide *back-bone* containing tetrahedral carbon atoms between amide groups. The peptide chain is obtained from condensation of amino acids: the amino group of one joins the carboxyl group of the next, forming a peptide bond.
   "Pharmaceutically acceptable" is meant to encompass any substance, which does not interfere with the effectiveness of the biological activity of the active ingredient and that is not toxic to the host to which is administered.
   "Polymorph" refers to a substance having the ability of assuming several crystalline forms in its solid state
   "Proteins" refers to a molecule comprising a polypeptide amino acid sequence. The main distinction between peptides and proteins is one of size. According to the present invention peptides contain not more than 100 amino acids, whereas proteins contain more than 100 amino acids.
   "PUK" refers to Pro-urokinase
   "Saccharide" refers to an aldehyde group or a ketone group having at least two hydroxyl groups, said saccharide adopting several forms: monomer form (monosaccharide), dimer form (disaccharide), trimer form (trisaccharide), oligomer (oligosaccharide) and polymer (polysaccaharide).
   "SOD" refers to Superoxide Dismutase
   "Surfactant" refers to an amphiphilic compound able to stabilize emulsions and suspensions of non-polar material in aqueous solution.
   "Co-surfactant" refers to an amphiphatic compound able to complete and optimize the action of the surfactant.
   "Therapeutically effective amount" refers to an amount that is sufficient to affect the course and the severity of the diseases described above, leading to the reduction or remission of such pathology. The effective amount will depend on the route of administration and the condition of the patient.
   "TNF" refers to Tumor Necrosis Factor
   "tPA" refers to Tissue Plasminogen Activator
   "UK" refers to urokinase
   "w/w" refers to weight/weight.

The present invention shall be illustrated by means of some examples which make references to the following Figures.

### Description of the Figures:

Figure 1: This figure relates to surface tension of Antide water solutions at different drug concentrations.
Figure 2: The scheme of said Figure 2 describes the method of production of peptide-loaded lipid nanoparticles according to the invention.
Figure 3:This figure shows LD frequency curves of an lipid nanoparticles formulation with the following composition: Antide 0.2%, Compritol E ATO 9.8%, Lutrol F68 5%, water up to 100%.

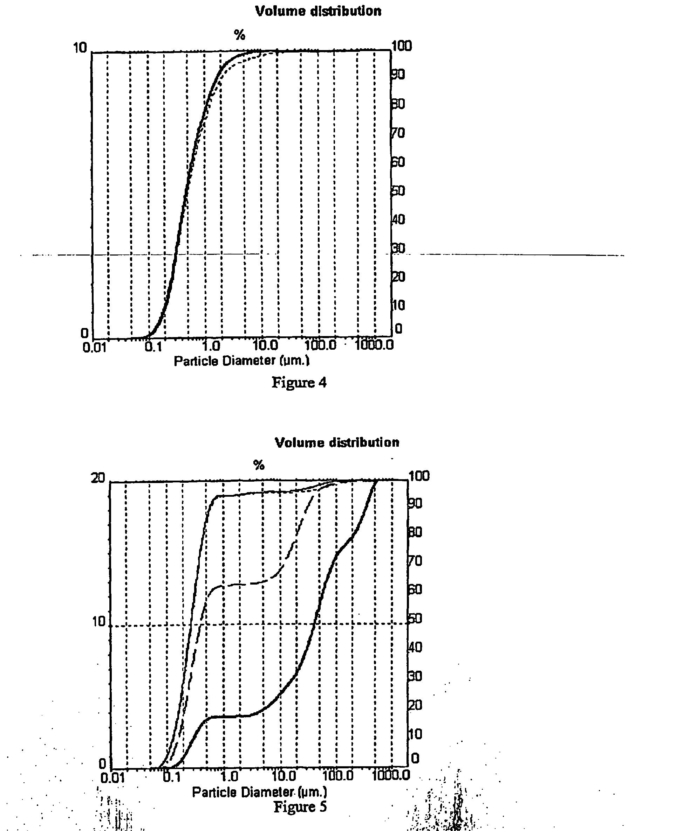

Figures 4 and 5: These figures show LD frequency curves of two different LN formulations loaded with cyclosporin. The first one (Figure 4) according to the invention is composed of Cyclosporin 0,2%, Compritol E ATO 9,3%, DMPG Na 0,5%, Tagat S 2,5% and Na cholate 0,5%.
   The second one (Figure 5), such as described in DE19819273, is composed of Cyclosporin 0,2%, Imwitor 900 9,3%, Tagat S 2,5% and Na cholate 0,5%. These measures have been performed for different t values:
   ..... t = 0;
   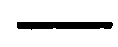 t = 24 h
   ------- t = 14 days
   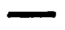 t = 1 month
Figure 6:This figure shows overlayed LD volume undersize curves of a lipid nanoparticles formulation (composed of Antide 0.2%, Compritol E ATO 9.3%, DMPG 0.5%, Tagat S 2.5%, Sodium Cholate 0.5%, water up to 100%), obtained from LD analysis at different times within 6 months.
Figure 7: This figure relates to DSC analyses of pure Compritol E ATO (monoglyceride content: 80%) and Imwitor 900 (monoglyceride content: 40-50%).
Figure 8: This figure relates to in vitro drug release profile in water from Antide-loaded LN27 and LN28.
   LN27 = Antide 0.2%, Compritol E ATO 9.3%, DMPG 0.5%, Tagat S 2.5%, Sodium Cholate 0.5%
   LN28 = Antide 0.2%, Compritol E ATO 9.3%, DMPG 0.5%, Lutrol F68 5%
   Water content in all formulations = up to 100%.
Figure 9: This figure relates to the assessment of the in vitro bioactivity of Antide incorporated in lipid systems. LN27 = Antide 0.2%, Compritol E ATO 9.3%, DMPG 0.5%, Tagat S 2.5%, Sodium Cholate 0.5%
   LN28 = Antide 0.2%, Compritol E ATO 9.3%, DMPG 0.5%, Lutrol F68 5%
   LN29 = Antide 0.2%, Compritol E ATO 9.8%, Lutrol F68 5%
   Water content in all formulations = up to 100%.
Figure 10: This figure shows the release kinetic of three different Antide-loaded lipid nanoparticles formulations when injected in rats subcutanelusly. The curves represents the plasma concentration of Antide during the time.

### EXAMPLES

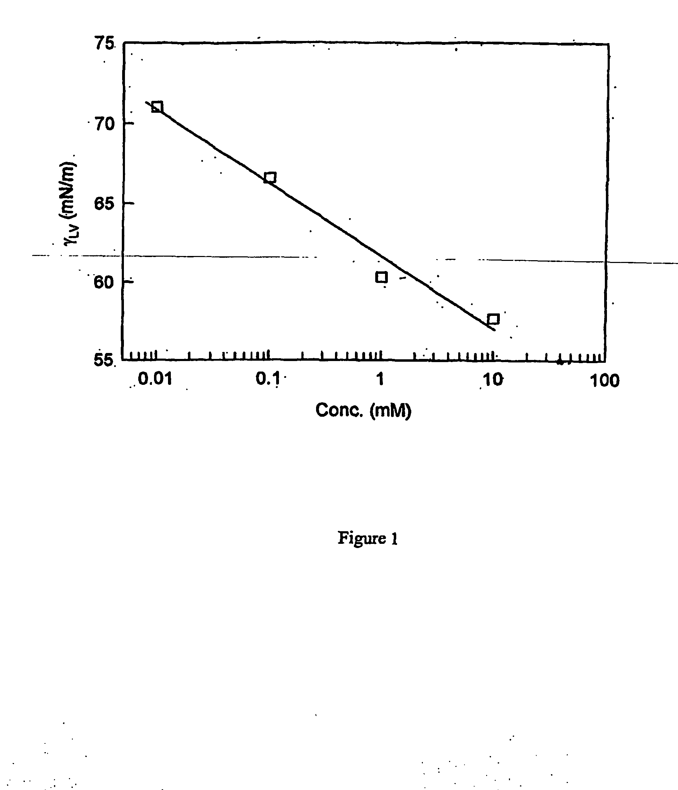

The peptide used in the Examples reported here below is Antide. This peptide has amphiphilic characteristics, as demonstrated by the following data:
Surface tension analysis: surface tension γ_{LV}, measurement was carried out using a Kruss tensiometer (drop shape analysis system) on Antide water solutions at different drug concentrations, namely 0.01, 0.1, 1.0, 10 mM. The results are shown in Figure 1.
Partition coefficient: it was determined using octanol as organic phase and water as hydrophilic phase. The two phases were first saturated with each other for 24 hours at room temperature. Antide was then dissolved in the water phase at a concentration well below saturation. An equal volume of organic phase was subsequently added to the water phase and the mixture was kept under stirring for 24 hours at room temperature. Antide concentration in the two phases was determined by RP-HPLC and the partition coefficient was obtained from the ratio between the drug concentration in organic and water phase.

The resulting octanol/water coefficient was 8.56 - 10⁻²

The results of Antide semi-quantitative solubility evaluation in some lipids, along with the lipid monoglyceride content are shown in Table 1.

### Preparation and characterization of pentide-loaded lipid nanoparticle

Some examples pertaining to the current invention are described below using different compositions. Figure 2 schematizes the lipid nanoparticles preparation by HPH method.

### Materials and equipment

Antide bulk, Bachem.
Imwitor 900 (Glyceryl monostearate), Condea Chemie-DE.
Compritol E ATO (Glyceryl monobehenate), Gattefossé-FR.
Compritol 888 ATO (Glyceryl behenate), Gattefossè-FR.
Imwitor 312 (Monoglyceride of lauric acid), Condea Chemie-DE.
Imwitor 928 (Glyceryl mono-/di-cocoate), Condea Chemie-DE.
Geleol (Glyceryl mono-palmitate/stearate), Gattefossè-FR.
Compritol HD 5 ATO (Glyceryl/polyethylene glycol behenate), Gattefossè-FR.
Superpolystate (Polyethylene glycol stearate), Gattefossè-FR.
Precirol ATO 5 (Glyceryl mono-/di-/tri-palmitate/stearate), Gattefossè-FR.
Witepsol E 85 (Tri-glycerides of C₁₀-C₁₈ saturated fatty acids), Massa Witepsol
Softisan 142 (Hydrogenated coco-glycerides), Condea Chemie-DE.
Gelot 64 (Glyceryl/polyethylene glycol palmitate/stearate), Gattefossè-FR.
Monosteol (Palmitate/stearate of propylene glycol), Gattefossè-FR.
Gelucire 44/14 (Defined blend of mono-/di-/tri-esters of lauric acid with glycerol and
polyethylene glycol), Gattefossè-FR.
Gelucire 50/13 (Defined blend of mono-/di-/tri-esters of stearic acid with glycerol and
polyethylene glycol), Gattefossè-FR.
Cetil alcohol, Sigma
Lutrol F68 (polyoxyethylen-polyoxypropylene block copolymer), Basf-D.
Tagat S (PEG30-glycerylstearate), Goldschmidt-D.
Capric acid, Sigma
Cholic acid sodium salt, ICN Aurora Ohio-USA.
1,2-dimyristoyl-phosphatidylglycerol (DMPG) sodium salt, Chemi-I.
1,2-dimyristoyl-phosphatidylcholine (DMPC), Chemi-I.
Noveon AA1 (Goodrich)
Pemulen TR-2 NF (Goodrich)

Perkin Elmer Pyris 1 Differential Scanning Calorimeter. The DSC analyses were performed in both heating and cooling mode with the following operative conditions: Range: 8 °C - 100 °C ; Scan rate: 5 °C/min; Pan (holed) capacity: 50µL; N₂ flux: 20µL/min. DSC parameters were evaluated on the 2^{nd} heating run evidencing the major thermal transitions.
Malvern Mastersizer Microplus MAF 5001 Laser Diffractometer.
Malvern Zetasizer 3000HS, (Size analysis and Zeta-potential analysis).
High Pressure Homogenizer MICRON LAB 40 (APV), equipped with a thermostatic jacket Drop shape analysis system DSA 10 - Krüss
Waters HPLC system: 2690 Separation Module; RP column, Jupiter 5 µm C18 (250 x 4.6 mm, 5 µm); 2487 Dual λ Absorbance Detector

### Example 1: Preparation of lipid nanoparticles using Compritol E ATO, Tagat S and Sodium cholate

After dispersing the peptide (Antide, 0.08 g) in the molten lipid (Compritol E ATO, 3.92 g) at 85°C, the warm aqueous phase with surfactant (Tagat S, 1.0 g) and co-surfactant (Sodium cholate, 0.2 g) was added. The obtained mixture was pre-homogenized, at the same temperature, using an ordinary homogenization tool, in order to obtain a pre-emulsion. Said emulsion was submitted to High Pressure Homogenization at a temperature between 80°C and 90°C. The so-obtained nanoemulsion was cooled down at controlled temperature conditions, and solid nanoparticles formed after crystallization of the lipid. The batch size was 40 g.

### Example 2: Preparation of lipid nanoparticles using Compritol E ATO, DMPG, Tagat S and Sodium cholate (LN27)

After dispersing the peptide (Antide, 0.08 g) in the molten lipid (Compritol E ATO, 3.72 g) containing DMPG (0.2 g) at 85°C, the warm aqueous phase with surfactant (Tagat S, 1.0 g) and co-surfactant (Sodium cholate, 0.2 g) was added. The obtained mixture was pre-homogenized, at the same temperature, using an ordinary homogenization tool, in order to obtain a pre-emulsion. Said emulsion was submitted to High Pressure Homogenization at a temperature between 80°C and 90°C. The so-obtained nanoemulsion was cooled down at controlled temperature conditions, and solid nanoparticles formed after crystallization of the lipid. The batch size was 40 g.

### Example 2A: Preparation of lipid nanoparticles using Imwitor 900, DMPG, Tagat S and Sodium cholate

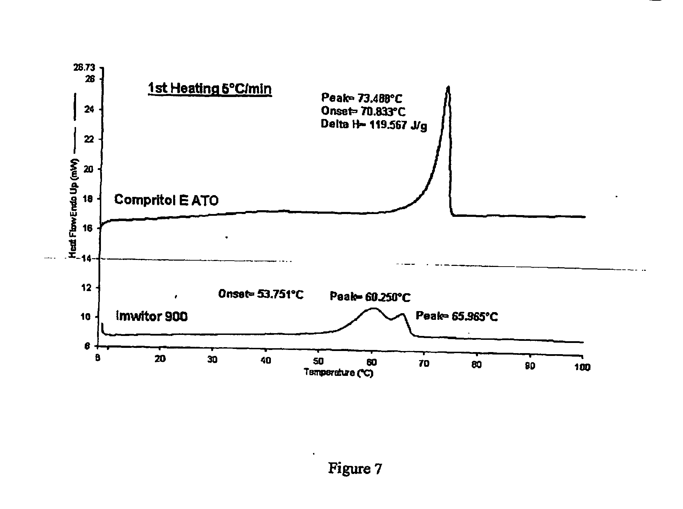

Example 2 was reapeated, but using Imwitor 900 instead of Compritol E ATO. At the end of the procedure the nanoparticles did not form because the particles tended to aggregate.This is due to the differences in monoglyceride content, melting peaks and crystalline content between the two lipids. All such differences have been reported in Figure 7.

### Example 3: Preparation of lipid nanoparticles using of Compritol E ATO, Imwitor 900, DMPG, Tagat S and Sodium cholate.

After dispersing the peptide (Antide, 0.08 g) in the molten lipid blend (Compritol E ATO and Imwitor 900, 9:1, 3.72 g) containing DMPG (0.2 g) at 85°C, the warm aqueous phase with surfactant (Tagat S, 1.0 g) and co-surfactant (Sodium cholate, 0.2 g) was added. The obtained mixture was pre-homogenized, at the same temperature, using an ordinary homogenization tool, in order to obtain a pre-emulsion. Said emulsion was submitted to High Pressure Homogenization at a temperature between 80°C and 90°C.

The so-obtained nanoemulsion was cooled down at controlled temperature conditions, and solid nanoparticles formed after crystallization of the lipid. The batch size was 40 g.

### Example 4: Preparation of lipid nanoparticles using of Compritol E ATO, DMPG, and LUTROL F68 (LN28)

After dispersing the peptide (Antide, 0.08 g) in the molten lipid (Compritol E ATO, 3.72 g) containing DMPG (0.2 g) at 85°C, the warm aqueous phase with surfactant (Lutrol F68, 2.0 g) was added. The obtained mixture was pre-homogenized, at the same temperature, using an ordinary homogenization tool, in order to obtain a pre-emulsion. Said emulsion was submitted to High Pressure Homogenization at a temperature between 80°C and 90°C. The so-obtained nanoemulsion was cooled down at controlled temperature conditions, and solid nanoparticles formed after crystallization of the lipid. The batch size was 40 g.

### Example 5: Preparation of lipid nanoparticles using of Compritol E ATO and Lutrol F68 (LN29)

After dispersing the peptide (Antide, 0.08 g) in the molten lipid (Compritol E ATO, 3.92 g) at 85°C, the warm aqueous phase with surfactant (Lutrol F68, 2.0 g) was added. The obtained mixture was pre-homogenized, at the same temperature, using an ordinary homogenization tool, in order to obtain a pre-emulsion. Said emulsion was submitted to High Pressure Homogenization at a temperature between 80°C and 90°C. The so-obtained nanoemulsion was cooled down at controlled temperature conditions, and solid nanoparticles formed after crystallization of the lipid. The batch size was 40 g.

### Example 6: Preparation of lipid nanoparticles using of Compritol E ATO, Capric acid Tagat S and Sodium cholate

After dispersing the peptide (Antide, 0.08 g) in the molten lipid phase (Compritol E ATO, 3.64 g and capric acid, 0.08 g) at 85°C, the warm aqueous phase with surfactant (Tagat S, 1.0 g) and co-surfactant (Sodium cholate, 0.2 g) was added. The obtained mixture was pre-homogenized, at the same temperature, using an ordinary homogenization tool, in order to obtain a pre-emulsion. Said emulsion was submitted to High Pressure Homogenization at a temperature between 80°C and 90°C. The so-obtained nanoemulsion was cooled down at controlled temperature conditions, and solid nanoparticles formed after crystallization of the lipid. The batch size was 40 g.

### Example 7: Preparation of lipid nanoparticles using of Compritol E ATO, DMPG, Lutrol F68 and Noveon AA1

The peptide (Antide, 0.08 g) was dispersed in the molten lipid (Compritol E ATO, 3.92 g) containing DMPG (0.4 g) at 85°C. The aqueous phase was prepared as follows: the surfactant (Lutrol F68, 2.0 g) was dissolved in half of the aqueous phase volume (about 17 mL of water), while Noveon AA1 (4 mg) was dispersed into the other half of the aqueous phase volume (about 17 mL of water). The warm aqueous phase with surfactant was first added to the molten lipid phase, and subsequently the other part of the aqueous phase containing Noveon was poured into the mixture, which was then pre-homogenized, at the same temperature, using an ordinary homogenization tool, in order to obtain a pre-emulsion. Said emulsion was submitted to High Pressure Homogenization at a temperature between 80°C and 90°C. The so-obtained nanoemulsion was cooled down at controlled temperature conditions, and solid nanoparticles formed after crystallization of the lipid. The batch size was 40 g.

### Example 8: Preparation of lipid nanoparticles using of Compritol E ATO, Lutrol F68 and Pemulen TR-2 NF

The peptide (Antide, 0.08 g) was dispersed into the molten lipid (Compritol E ATO, 3.92 g) containing DMPG (0.4 g) at 85°C. The aqueous phase was prepared as follows: the surfactant (Lutrol F68, 2.0 g) was dissolved in half of the aqueous phase volume (about 17 mL of water), while Pemulen (4 mg) was dispersed into the other half of the aqueous phase volume (about 17 mL of water). The warm aqueous phase with surfactant was first added to the molten lipid phase, and subsequently the other part of the aqueous phase containing Pemulen was poured into the mixture, which was then pre-homogenized, at the same temperature, using an ordinary homogenization tool, in order to obtain a pre-emulsion. Said emulsion was submitted to High Pressure Homogenization at a temperature between 80°C and 90°C. The so-obtained nanoemulsion was cooled down at controlled temperature conditions, and solid nanoparticles formed after crystallization of the lipid. The batch size was 40 g.

The characterization of the lipid nanoparticles prepared as described in Examples 1-5 is reported below.

### Example 9: Evaluation of Antide encapsulation efficiency into lipid nanoparticles:

Antide encapsulation efficiency into lipid nanoparticles was determined by RP-HPLC. The lipid nanoparticles suspension was first completely dissolved in acetone. Antide was then extracted adding water to the organic phase. The mixture of two phases was stirred, sonicated and centrifuged, and the clear phase was subsequently withdrawn and injected into the HPLC column. It should be noticed that the drug content determined by this method has to be considered as a total drug content, without any reference to Antide included in the lipid nanoparticles or dissolved in the continuous aqueous phase. Surprisingly, said Antide was encapsulated with an efficiency of 85-90 %. Moreover the lipid nanoparticles formed were physically and chemically stable at 4°C over at least 6 months after preparation. As a matter of fact no degradation of the active ingredient has been found and the physical characteristics of the lipid nanoparticles were unchanged; no aggregation between the particles has been noted.

### Example 10: In-vitro Antide release:

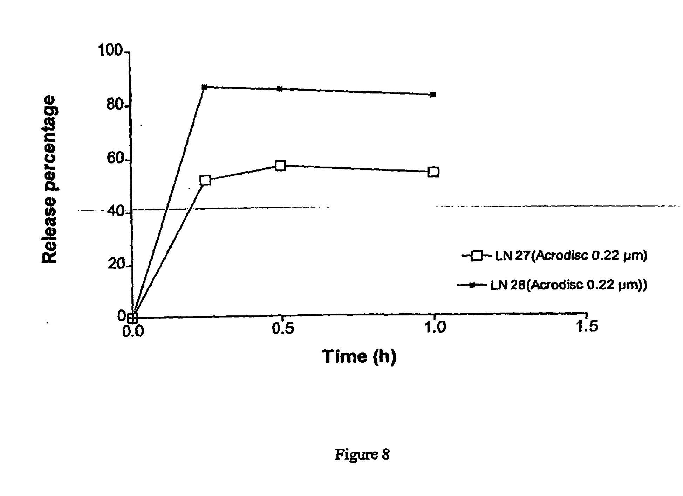

Antide-lipid nanoparticles were incubated into the release medium (water) and samples were withdrawn at different times, and subsequently filtered through 0.22 µm Acrodisc filters. The clear solution was analyzed by RP-HPLC. The results of the Antide release from two formulations are shown in Figure 8.

### Example 11: lipid nanoparticles physico-chemical characterization:

the particle size and particle size distribution of Antide-loaded lipid nanoparticles formulation were evaluated by Laser Diffractometry (LD) and Photon Correlation Spectroscopy (PCS), using the Malvern's Laser Diffractometer and the Zetasizer respectively. The Zeta potential of the lipid nanoparticles was also measured, using the Zetasizer. Results are reported in Table 3. For LD analysis, few drops of lipid nanoparticles were dispersed in 100 mL of deionized water, so to obtain an obscuration value between 5% and 30%. The sample was kept circulating within the dispersion unit at a pump speed of 1500 rpm. At least three measurements were taken for each sample and the data were processed using the presentation 5NFD (particle RI=[1.456, 0.01] as real and imaginary parts respectively, and dispersant RI=1.3300). PCS-size analysis was carried out using filtered (0.2 µm) MilliQ water as dispersing medium. The concentration of lipid nanoparticles formulation used for the analysis was about 1 µL/mL, so to obtain a count rate value below 500 Kcps. For Z-Potential analysis of the lipid nanoparticles a filtered (0.2 µm) NaCl aqueous solution with a conductivity of about 50 µS (exactly measured by a Conductivity Meter) was used as dispersant. The lipid nanoparticles formulation concentration for this analysis was about 0.3 µL/mL, so to obtain a count rate of around 3000-3500 Kcps.

### BIOLOGICAL RESULTS

### Example 12: In vitro assay

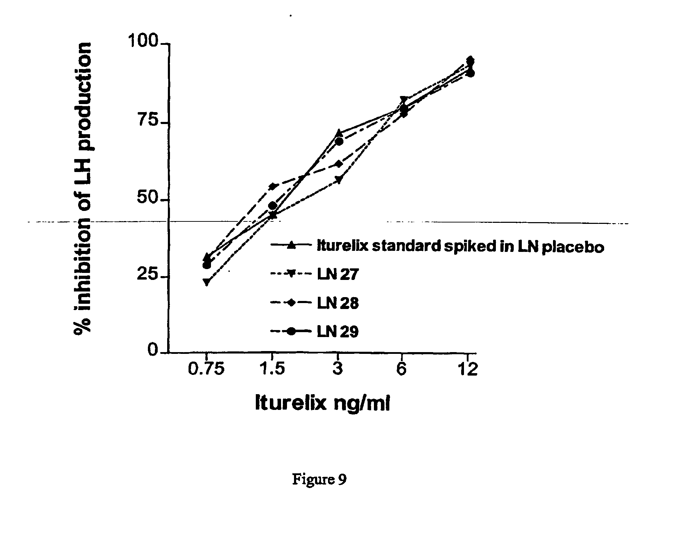

The results shown in Figure 9 confirm that lipid nanoparticles preparation method did not cause any major modification of drug activity. This has been shown in the rat pituitary cell assay, carried out as described here below.

Primary culture of rat pituitary cells was established starting from enzymatic digestion of pituitary glands removed from female rats. Recovered cells were plated at 2.5x10⁵/well in 24-well plates and cultured for 72 hours at 37°C and 5% CO₂.

Wells were washed three times and then treated for 24 hours with 0.75, 1.5, 3, 6, and 12 ng/ml of three lipid nanoparticles-Antide formulations (LN27, LN28 and LN29) or in house reference standard Antide in triplicate. Wells for basal and maximum level of secreted LH received culture medium alone.

Then, after washing, samples and reference Antide dilutions were renewed and LHRH (10⁻⁸M) was added in all the wells except to the basal wells that received equal volume of culture medium. Conditioned medium from each well was collected after 4 hours of incubation (37°C, 5% CO₂) and stored at -20°C until assayed for LH content.

For the evaluation of secreted LH, a commercial RIA (Amersham Pharmacia Biotech) was used. Results were expressed as percentage inhibition of LH secretion by Antide.

### Example 13: in vivo assay

Adult (63-70 days and about 300 g) Sprague Dawley male rats have been used in the study. The diet was available "ad libitum" to all the animals. The drinking water was also offered to the animals "ad libitum".

The test formulations containing lipid nanoparticles of Antide have been administered as one single subcutaneous dose of 0.6 mg (about 2 mg/kg) as Antide to each group of rats by subcutaneous route. Lipid nanoparticles of Antide have been administered in an approx. 5% glucose aqueous solution.

The nanoparticles contents in the vehicle was about 120 mg/ml. The volume of administration was 400 µl per rat
The following experimental design was followed:

| | ***Groups*** | | | | | | | |
|---|---|---|---|---|---|---|---|---|
| | 1 | 2 | 3 | 4 | 5 | 6 | 7 | 8 |
| Test article | Antide | Antide LN27 | Antide LN28 | Antide LN29 | Antide LN271 | Antide LN28 | Antide LN29 | Placebo n-particles |
| Antide dose (mg) | 0.6 | 0.6 | 0.6 | 0.6 | 0.6 | 0.6 | 0.6 | 0 |
| No. rats/ group | 3 | 3 | 3 | 3 | 30 | 30 | 30 | 12 |
| Blood sampling | 0.5, 1, 2, 4, 8, 24, 48, 72 h | 0.5, 1, 2, 4, 8, 24 h | 0.5, 1, 2, 4, 8, 24 h | 0.5, 1, 2, 4, 8, 24 h | 0, 2, 3, 4, 5, 6, 8, 10, 12, 14 days | 0, 2, 3, 4, 5, 6, 8, 10, 12, 14 days | 0, 2, 3, 4, 5, 6, 8, 10, 12, 14 days | 1, 4, 8, 14 days |

The compounds have been administered to the animals which were fasted overnight prior to administration.

From animals of groups 1-4 about 0.5-1 ml of blood was drawn from a sublingual or tail vein at each sampling time up to 8 hours. At 24 hours (72 hours for group 1) the animals were aneasthetized with ether and killed by exsanguination from the abdominal aorta.

Animals of groups 5-8 were sampled by exsanguination from the abdominal aorta at the indicated sampling times.

Blood was collected in heparinized tubes and plasma separated by centrifugation (2500 x g) at 4°C. Plasma obtained at sacrifice was divided into 3 aliquots of at least 1 ml.

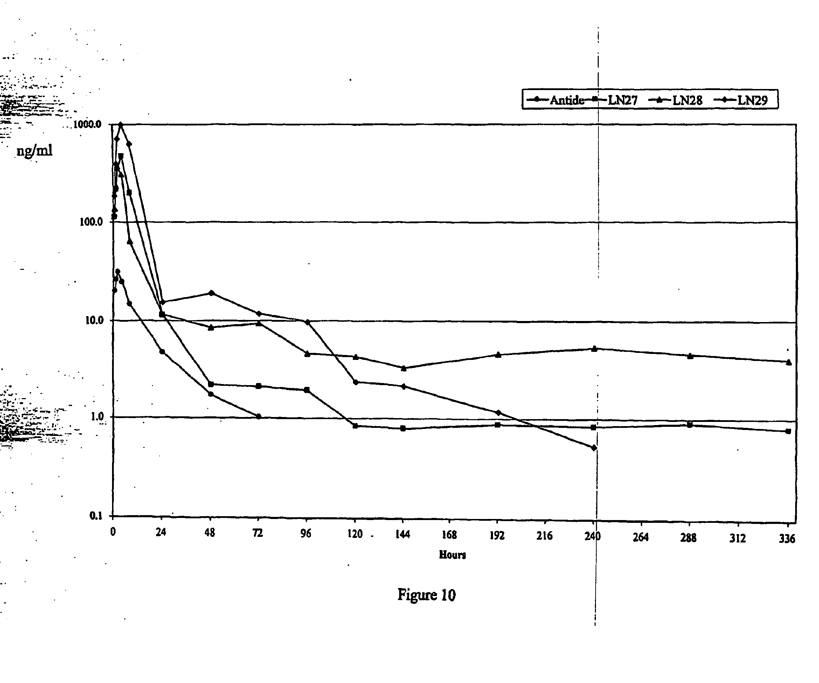

The plasma concentrations of Antide was determined by an HPLC method with Mass Spectrometry detection (HPLC/MS/MS). Such pK results are shown in Figure 10.

The pharmacodynamic marker testosterone was measured in all plasma samples taken at sacrifice.

Testosterone levels were determined using a RIA kit from Diagnostic Product Corporation (DPC).

The results obtained are reported in Table 4, which shows that testosterone production was actually inhibited for a short period after administration, indicating a sustained release of Antide. Moreover, the 0.0 ± 0.0 of testosterone plasma concentration obtained one day after administration really indicates a total suppression of testosterone levels.

**Table 1: Antide maximum loading in the pre-screened lipids, and monoglyceride content of the lipids.**

| **LIPIDS** | | | **Loading** |
|---|---|---|---|
| **Product** | **Chemical description** | **Monoglyceride content (%)** | **Antide loading (%)** |
| Imwitor 312 | Monoglyceride of lauric acid | 95.30 | 2.2 |
| Imwitor 900* | Glyceryl mono-/di-stearate | 40-50 | 2.0 |
| Imwitor 928 | Glyceryl mono-/di-cocoate | 43.5 | 8.5x10⁻¹ |
| Geleol | Glyceryl mono-palmitate/stearate | 35 | 1.8 |
| Compritol E ATO | Glyceryl mono-/di-/tri-behenate | 80.40 | 1.7 |
| Compritol 888 ATO | Glyceryl behenate | 12-18 | 4.3x10⁻¹ |
| Compritol HD 5 ATO | Glyceryl/polyethylene glycol behenate | 1 | 1,7x10⁻² |
| Superpolystate | Polyethylene glycol stearate | <1 | 1.7x10⁻¹ |
| Precirol ATO 5 | Glyceryl mono-/di-/tri-palmitate/stearate | 8-17 | 5.9x10⁻² |
| Witepsol E 85 | Trlglycerides of C₁₀-C₁₈ saturated fatty acids | <1 | 1.4x10⁻² - not soluble |
| Softisan 142 | Hydrogenated coco-glycerides | <1% | 1.7x10⁻² - not soluble |
| Gelot 64 | Glyceryl/polyethylene glycol palmitate/stearate | <1% | 5.8x10⁻² - not soluble |
| Monosteol | Palmitate/stearate of propylene glycol | <1% | 3.2x10⁻² - not soluble |
| Gelucire 44/14 | Defined blend of mono-/di-/tri-esters of lauric acid with glycerol and polyethylene glycol | <1% | 4.0x10⁻² - not soluble |
| Gelucire 50/13 | Defined blend of mono-/di-/tri-esters of stearic acid with glycerol and polyethylene glycol | <1% | 3.0x10⁻² not soluble |
| Cetil alcohol | Cetil alcohol | <1% | 3.7x10⁻² - not soluble |
| Tagat S | | <1% | 2.7x10⁻² - not soluble |

**Table 2: Major thermal transitions of lipid/lipid blends (the numbers in brackets in the second column refer to secondary thermal transitions).**

| **LIPIDS** | **Melting Peaks (°C)** |
|---|---|
| Compritol 888 ATO | 72.0 |
| Compritol E ATO | 73.7 |
| Imwitor 900 | 58.6 |
| Imwitor 312 | (21.6), (42.5), 56.3 |
| Precirol ATO 5 | (49.0), 57.2 |
| Compritol 888 ATO + Imwitor 900 [75:25] | 68.7 |
| Compritol 888 ATO + Imwitor 900 [50:50] | (58.7), 64.5 |
| Compritol 888 ATO + Imwitor 900 [25:75] | 61.2 |
| Compritol 888 ATO + Imwitor 312 [75:25] | (37.1),66.8 |
| Compritol 888 ATO + Imwitor 312 [50:50] | (37.3), 51.3, 64.3 |
| Compritol 888 ATO + Imwitor 312 [25:75] | (22.2), (38.7), 53.7, (60.4) |
| Compritol 888 ATO + Precirol ATO 5 [75:25] | (56.4), 68.5 |
| Compritol 888 ATO + Precirol ATO 5 [50:50] | 56.6, 64.3 |
| Compritol 888 ATO + Precirol ATO 5 [25:75] | (49.0), 58.8 |
| Imwitor 900 + Imwitor 312 [75:25] | 53.5 |
| Imwitor 900 + Imwitor 312 [50:50] | (38), 46.4 |
| Imwitor 900 + Imwitor 312 [25:75] | (32.4), 40.0 |
| Imwitor 900 + Precirol ATO 5 [75:25] | 59.2 |
| Imwitor 900 + Precirol ATO 5 [50:50] | (49.2), 57.9 |
| Imwitor 900 + Precirol ATO 5 [25:75] | (49.0), 57.9 |
| Precirol ATO 5 + Imwitor 312 [75:25] | 51.5 (wide) |
| Precirol ATO 5 + Imwitor 312 [50:50] | 38.2, 47.9 |
| Precirol ATO 5 + Imwitor 312 [25:75] | (32.2), 39.3 |

**Table 3: Physico-chemical characterization of lipid nanoparticles batches with different compositions.**

| **Ex. N.** | **Composition (% w/w)** | | **Operating conditions** | **D (v,0.1) (µm)** | **D (v,0.5) (µm)** | **D (v,0.9) (µm)** | **PCS size (nm)** | **Z-pot (mV)** |
|---|---|---|---|---|---|---|---|---|
| 1 | Antide | 0.2 | T = 85°C | | | | | |
| | Compr. E ATO | 9.8 | p = 1000 bar | 0.17 | 0.57 | 2.83 | 278 | -14.4 |
| | Tagat S | 2.5 | 1 cycle | | | | | |
| | Chol.Na | 0.5 | | | | | | |
| 2 | Antide | 0.2 | | | | | | |
| | Compr. E ATO | 9.3 | T = 85°C | | | | | |
| | DMPG | 0.5 | p = 1000 bar | 0.18 | 0.52 | 5.21 | 274 | -38.9 |
| | Tagat S | 2.5 | 1 cycle | | | | | |
| | Chol.Na | 0.5 | | | | | | |
| 3 | Antide | 0.2 | | | | | | |
| | Comp.E ATO⁺ | | T = 85°C | | | | | |
| | Imw 900 (9:1) | 9.3 | p =1000 bar | | | | | |
| | DMPG | 0.5 | 1 cycle | 0.17 | 0.48 | 3.42 | 233 | -48.9 |
| | Tagat S | 2.5 | | | | | | |
| | Chol.Na | 0.5 | | | | | | |
| 4 | Antide | 0.2 | T = 85°C | 0.19 | | | | |
| | Compr. E ATO | 9.3 | p =1000 bar | | | | | |
| | DMPG | 0.5 | 1 cycle | | 0.54 | 4.36 | 242 | -39.4 |
| | Lutrol F68 | 5.0 | | | | | | |
| 5 | Antide | 0.2 | T = 85°C | 0.19 | 0.46 | 1.84 | | |
| | Compr. E ATO | 9.8 | p = 1000 bar | | | | N/A | N/A |
| | Lutrol F68 | 5.0 | 1 cycle | | | | | |
| 6 | Antide | 0.2 | | | | | | |
| | Compr. E ATO | 9.1 | T = 85°C | | | | | |
| | Capric acid | 0.2 | p = 1000 bar | 0.15 | 0.38 | 3.07 | 149 | -38.5 |
| | DMPG | 0.5 | 1 cycle | | | | | |
| | Tagat S | 2.5 | | | | | | |
| | Chol.Na | 0.5 | | | | | | |
| 7 | Antide | 0.2 | | | | | | |
| | Compr. E ATO | 9.8 | T = 85°C | | | | | |
| | DMPG | 1.0 | p = 1000 bar | 0.17 | 0.41 | 5.55 | 160 | -48.8 |
| | Lutrol F68 | 5.0 | 1 cycle | | | | | |
| | Noveon AA1 | 0.01 | | | | | | |
| 8 | Antide | 0.2 | | | | | | |
| | Compr. E ATO | 8.8 | T = 85°C | | | | | |
| | DMPG | 1.0 | p = 1000 bar | 0.18 | 0.46 | 6.66 | 189 | -50.5 |
| | Lutiol F68 | 5.0 | 1 cycle | | | | | |
| | Pemulen | 0.01 | | | | | | |

**Table 4. Testosterone plasma levels at t=0, t=1 day and t= 8 days after s.c. administration in rats (testosterone plasma concentration mean values and corresponding standard deviation (SD) in ng/mL are shown).**

| **Time (days)** | Testosterone plasma levels (ng/mL) | | | | | | | |
|---|---|---|---|---|---|---|---|---|
| | LN27 | | LN28 | | LN29 | | Placebo | |
| | Mean | SD | Mean | SD | Mean | SD | Mean | SD |
| **0** | 3.3 | 3.4 | 2.2 | 1.2 | 3.1 | 4.6 | N/A | N/A |
| **1** | 0.0 | 0.0 | 0.0 | 0.0 | 0.0 | 0.0 | 3.4 | 2.3 |
| **8** | 1.2 | 0.8 | 1.7 | 0.7 | 0.5 | 0.4 | 4.2 | 4.9 |

## Claims

1. Lipid nanoparticles comprising a drug, a lipid matrix and a surfactant **characterized in that** said drug is a peptide or a protein and said lipid matrix has a monoglyceride content which is at least 70% w/w, the percentage being based on the weight of the lipid matrix, wherein said nanoparticles are in a range from 1 nm to 3 ,000 nm.

2. Lipid nanoparticles according to claim 1 **characterized in that** said monoglyceride content is comprised between 75 and 99% w/w, the percentage being based on the weight of the lipid matrix.

3. Lipid nanoparticles according to claim 1 or 2 **characterized in that** said lipid nanoparticles also comprise a co-surfactant.

4. Lipid nanoparticles according to claim 1 **characterized in that** said drug is a protein.

5. Lipid nanoparticles according to claim 1 **characterized in that** said drug is a peptide.

6. Lipid nanoparticles according to claim 1 **characterized in that** said drug is a peptide selected from the group consisting of LHRH analogs.

7. Upid nanoparticles according to claim 6 **characterized in that** said peptide is a decapeptide acting as LHRH antagonist.

8. Lipid nanoparticles according to claim 6 or 7 **characterized in that** said decapeptide is, N-Ac-D-2-Nal -D-pClPhe -D-3-Pal-Ser- NicLys- D-NicLys-Leu-Ilys -Pro -D-Ala -NH₂.

9. Lipid nanoparticles according to claim 6 or 7 **characterized in that** said decapeptide is cetrorelix.

10. Lipid nanoparticles according to any of preceding claims **characterized in that** said surfactant is selected among synthetic phospholipids, their hydrogenated derivatives and mixtures thereof, sphingolipids and glycosphingolipids, saturated or unsaturated fatty acids, fatty alcohols, polyoxyethylene-polyoxypropylene copolymers, ethoxylated fatty acids as well as esters or ethers thereof, or a combination of two or more of the above mentioned.

11. Lipid nanoparticles according to claim 10 **characterized in that** said surfactant is dimyristoyl phosphatidyl glycerol.

12. Lipid nanoparticles according to claim 3 **characterized in that** said co-surfactant is selected from the group consisting of butanol, butyric acid, hexanoic acid, sodium cholate, sodium taurocholate or sodium glycocholate.

13. Lipid nanoparticles according to any of the preceding claims **characterized in that** said nanoparticles also comprise other pharmaceutically acceptable excipients.

14. Lipid nanoparticles according to claim 13 **characterized in that** such excipients are polymers with bioadhesive or absorption enhancing properties selected from the group consisting of acrylic polymers, medium chain fatty acids and polyethylene glycols.

15. Lipid nanoparticles according to any of the preceding claims for use as a medicament.

16. A pharmaceutical composition containing lipid nanoparticles according to any of the preceding claims and a pharmaceutically acceptable carrier, diluent or excipient.

17. Process for the production of lipid nanoparticles according to any of claims 1-15 comprising or consisting of the steps of:
- incorporation of the drug into the lipid phase and dissolution of the surfactant and optionally the co-surfactant in the aqueous phase, under controlled heating conditions
- mixing of the lipid phase with the aqueous phase
- applying a High Pressure Homogenization to the obtained pre-emulsion and
- cooling down the nano-emulsion under controlled temperature conditions.

18. Process according to claim 17 **characterized in that** said steps are carried out at a pH comprised between 1 and 9.

19. Process according to claim 17 **characterized in that** said mixing step between lipid and aqueous phase is carried out at a temperature comprised between 30°C and 90°C.

20. Process according to claim 17 **characterized in that** said High Pressure Homogenization step is carried out at a temperature comprised between 30°C and 90°C.

21. Process according to claim 17 **characterized in that** said High Pressure Homogenization step is carried out at a pressure comprised between 50 bar and 2000 bar.

22. Lipid nanoparticles obtained by a process according to any of claims 17-21

## Patentansprüche

1. Lipidnanopartikel, umfassend einen Wirkstoff, eine Lipidmatrix und ein oberflächenaktives Mittel, **dadurch gekennzeichnet, dass** der Wirkstoff ein Peptid oder ein Protein ist und die Lipidmatrix einen Monoglyceridgehalt hat, der mindestens 70% G/G ist, wobei der Prozentsatz auf dem Gewicht der Lipidmatrix basiert, wobei die Nanopartikel in einem Bereich von 1 nm bis 3000 nm sind.

2. Lipidnanopartikel gemäß Anspruch 1, **dadurch gekennzeichnet, dass** der Monoglyceridgehalt zwischen 75 und 99% G/G ausmacht, wobei der Prozentsatz auf dem Gewicht der Lipidmatrix basiert.

3. Lipidnanopartikel gemäß Anspruch 1 oder 2, **dadurch gekennzeichnet, dass** die Lipidnanopartikel auch ein oberflächenaktives Co-Mittel umfassen.

4. Lipidnanopartikel gemäß Anspruch 1, **dadurch gekennzeichnet, dass** der Wirkstoff ein Protein ist.

5. Lipidnanopartikel gemäß Anspruch 1, **dadurch gekennzeichnet, dass** der Wirkstoff ein Peptid ist.

6. Lipidnanopartikel gemäß Anspruch 1, **dadurch gekennzeichnet, dass** der Wirkstoff ein Peptid ist, ausgewählt aus der Gruppe, bestehend aus LHRH-Analoga.

7. Lipidnanopartikel gemäß Anspruch 6, **dadurch gekennzeichnet, dass** das Peptid ein Decapeptid ist, das als LHRH-Antagonist wirkt.

8. Lipidnanopartikel gemäß Anspruch 6 oder 7, **dadurch gekennzeichnet, dass** das Decapeptid N-Ac-D-2-Nal-D-pClPhe-D-3-Pal-Ser-NicLys-D-NicLys-Leu-Ilys-Pro-D-Ala-NH₂ ist.

9. Lipidnanopartikel gemäß Anspruch 6 oder 7, **dadurch gekennzeichnet, dass** das Decapeptid Cetrorelix ist.

10. Lipidnanopartikel gemäß einem der vorhergehenden Ansprüche, **dadurch gekennzeichnet, dass** das oberflächenaktive Mittel ausgewählt ist unter synthetischen Phospholipiden, ihren hydrierten Derivaten und Gemischen davon, Sphingolipiden und Glycosphingolipiden, gesättigten oder ungesättigten Fettsäuren, Fettalkoholen, Polyoxyethylen-Polyoxypropylen-Copolymeren, ethoxylierten Fettsäuren sowie Estern oder Ethern davon, oder einer Kombination aus zwei oder mehr der oben genannten.

11. Lipidnanopartikel gemäß Anspruch 10, **dadurch gekennzeichnet, dass** das oberflächenaktive Mittel Dimyristoylphosphatidylglycerin ist.

12. Lipidnanopartikel gemäß Anspruch 3, **dadurch gekennzeichnet, dass** das oberflächenaktive Co-Mittel ausgewählt ist aus der Gruppe, bestehend aus Butanol, Buttersäure, Hexansäure, Natriumcholat, Natriumtaurocholat oder Natriumglycocholat.

13. Lipidnanopartikel gemäß einem der vorhergehenden Ansprüche, **dadurch gekennzeichnet, dass** die Nanopartikel auch andere pharmazeutisch annehmbare Exzipientien umfassen.

14. Lipidnanopartikel gemäß Anspruch 13, **dadurch gekennzeichnet, dass** derartige Exzipientien Polymere mit bioadhäsiven oder absorptionserhöhenden Eigenschaften sind, ausgewählt aus der Gruppe, bestehend aus Acrylpolymeren, mittelkettigen Fettsäuren und Polyethylenglykolen.

15. Lipidnanopartikel gemäß einem der vorhergehenden Ansprüche zur Verwendung als Medikament.

16. Pharmazeutische Zusammensetzung, enthaltend Lipidnanopartikel gemäß einem der vorhergehenden Ansprüche, und einen pharmazeutisch annehmbaren Träger, ein pharmazeutisch annehmbares Verdünnungsmittel oder ein pharmazeutisch annehmbares Exzipiens.

17. Verfahren zur Herstellung von Lipidnanopartikeln gemäß einem der Ansprüche 1-15, umfassend die Stufen oder bestehend aus den Stufen:
- Einarbeitung des Wirkstoffs in die Lipidphase und Lösung des oberflächenaktiven Mittels und gegebenenfalls des oberflächenaktiven Co-Mittels in der wässrigen Phase unter kontrollierten Erwärmungsbedingungen,
- Mischen der Lipidphase mit der wässrigen Phase,
- Anwenden einer Hochdruckhomogenisierung auf die erhaltene Voremulsion und
- Abkühlen der Nanoemulsion unter kontrollierten Temperaturbedingungen.

18. Verfahren gemäß Anspruch 17, **dadurch gekennzeichnet, dass** die Stufen bei einem pH durchgeführt werden, der von zwischen 1 und 9 umfasst wird.

19. Verfahren gemäß Anspruch 17, **dadurch gekennzeichnet, dass** die Stufe des Mischens zwischen Lipid- und wässriger Phase bei einer Temperatur durchgeführt wird, die von zwischen 30°C und 90°C umfasst wird.

20. Verfahren gemäß Anspruch 17, **dadurch gekennzeichnet, dass** die Hochdruckhomogenisierungsstufe bei einer Temperatur durchgeführt wird, die von zwischen 30°C und 90°C umfasst wird.

21. Verfahren gemäß Anspruch 17, **dadurch gekennzeichnet, dass** die Hochdruckhomogenisierungsstufe bei einem Druck durchgeführt wird, der von zwischen 50 bar und 2000 bar umfasst wird.

22. Lipidnanopartikel, erhalten durch ein Verfahren gemäß einem der Ansprüche 17-21.

## Revendications

1. Nanoparticules lipidiques comprenant un médicament, une matrice lipidique et un tensioactif, **caractérisées en ce que** ledit médicament est un peptide ou une protéine et ladite matrice lipidique présente une teneur en monoglycérides d'au moins 70 %, en poids rapporté au poids total de la matrice lipidique, lesdites nanoparticules ayant une taille de 1 à 3000 nm.

2. Nanoparticules lipidiques conformes à la revendication 1, **caractérisées en ce que** ladite teneur en monoglycérides vaut entre 75 et 99 %, en poids rapporté au poids total de la matrice lipidique.

3. Nanoparticules lipidiques conformes à la revendication 1 ou 2, **caractérisées en ce que** ces nanoparticules lipidiques comprennent aussi un co-tensioactif.

4. Nanoparticules lipidiques conformes à la revendication 1, **caractérisées en ce que** ledit médicament est une protéine.

5. Nanoparticules lipidiques conformes à la revendication 1, **caractérisées en ce que** ledit médicament est un peptide.

6. Nanoparticules lipidiques conformes à la revendication 1, **caractérisées en ce que** ledit médicament est un peptide choisi dans l'ensemble des analogues de l'hormone LH-RH (hormone de libération de la lutéino-stimuline).

7. Nanoparticules lipidiques conformes à la revendication 6, **caractérisées en ce que** ledit médicament est un décapeptide qui joue le rôle d'un antagoniste de l'hormone LH-RH.

8. Nanoparticules lipidiques conformes à la revendication 6 ou 7, **caractérisées en ce que** ledit décapeptide est représenté par la formule :
N-Ac-D-2-Nal-D-pClPhe-D-3-Pal-Ser-NicLys-D-NicLys-Leu-Ilys-Pro-D-Ala-NH₂.

9. Nanoparticules lipidiques conformes à la revendication 6 ou 7, **caractérisées en ce que** ledit décapeptide est du cetrorelix.

10. Nanoparticules lipidiques conformes à l'une des revendications précédentes, **caractérisées en ce que** ledit tensioactif est choisi parmi les phospholipides synthétiques, leurs dérivés hydrogénés et les mélanges de ces composés, les sphingolipides et glycosphingolipides, les acides gras saturés ou insaturés, les alcools gras, les copolymères de type poly(oxyéthylène)-poly(oxypropylène), et les acides gras éthoxylés ainsi que leurs esters ou éthers, ainsi que les combinaisons de deux des composés cités ci-dessus ou plus.

11. Nanoparticules lipidiques conformes à la revendication 10, **caractérisées en ce que** ledit tensioactif est du dimyristoyl-phosphatidyl-glycérol.

12. Nanoparticules lipidiques conformes à la revendication 3, **caractérisées en ce que** ledit co-tensioactif est choisi dans l'ensemble formé par les butanol, acide butyrique, acide hexanoïque, cholate de sodium, taurocholate de sodium et glycocholate de sodium.

13. Nanoparticules lipidiques conformes à l'une des revendications précédentes, **caractérisées en ce que** ces nanoparticules comprennent aussi d'autres excipients pharmacologiquement admissibles.

14. Nanoparticules lipidiques conformes à la revendication 13, **caractérisées en ce que** lesdits excipients sont des polymères possédant des propriétés bioadhésives ou favorisant l'absorption, choisis dans l'ensemble formé par les polymères polyacryliques, les acides gras à chaîne moyenne et les polyéthylèneglycols.

15. Nanoparticules lipidiques conformes à l'une des revendications précédentes, conçues pour servir de médicament.

16. Composition pharmaceutique contenant des nanoparticules lipidiques conformes à l'une des revendications précédentes, ainsi qu'un véhicule, diluant ou excipient pharmacologiquement admissible.

17. Procédé de production de nanoparticules lipidiques conformes à l'une des revendications 1 à 15, lequel procédé consiste en les opérations suivantes ou les comporte :
- incorporer le médicament dans la phase lipidique et dissoudre le tensioactif et, le cas échéant, le co-tensioactif dans la phase aqueuse, dans des conditions de régulation du chauffage ;
- mélanger la phase lipidique avec la phase aqueuse ;
- soumettre la pré-émulsion ainsi obtenue à une homogénéisation sous haute pression ;
- et faire refroidir la nano-émulsion formée, dans des conditions de régulation de la température.

18. Procédé conforme à la revendication 17, **caractérisé en ce que** lesdites opérations sont effectuées à un pH situé entre 1 et 9.

19. Procédé conforme à la revendication 17, **caractérisé en ce que** ladite opération de mélange de la phase lipidique et de la phase aqueuse est effectuée à une température située entre 30 et 90 °C.

20. Procédé conforme à la revendication 17, **caractérisé en ce que** ladite opération d'homogénéisation sous haute pression est effectuée à une température située entre 30 et 90 °C.

21. Procédé conforme à la revendication 17, **caractérisé en ce que** ladite opération d'homogénéisation sous haute pression est effectuée sous une pression située entre 50 et 2000 bars.

22. Nanoparticules lipidiques obtenues selon un procédé conforme à l'une des revendications 17 à 21.
